# EUROPEAN PATENT SPECIFICATION

(11) **EP 0 806 432 B1**
(45) Date of publication and mention of the grant of the patent: **20.03.2002**
(21) Application number: 97107082.6
(22) Date of filing: 29.04.1997
(51) Int. Cl.: C07K 5/072, C07K 5/02

(54) **Glutathione monoester sulfonate**
Sulfonat von Glutathion-Monoester
Monoester de glutathion sous forme sulfonate

(30) Priority: 09.05.1996 JP 11474096
(43) Date of publication of application: 12.11.1997
(73) Proprietor: KYOWA HAKKO KOGYO CO., Ltd., Chiyoda-ku, Tokyo 100 (JP)
(72) Inventor: Kato, Sachiko, Kawachinagano-shi, Osaka 586 (JP); Chujo, Iwao, Izumisano-shi, Osaka 598 (JP); Ogasa, Takehiro, Sakai-shi, Osaka 591 (JP); Kasai, Masaji, Fujisawa-shi, Kanagawa 251 (JP); Mimura, Yukiteru, Sunto-gun, Shizuoka 411 (JP)
(74) Representative: Casalonga, Axel

(56) References cited:
- WO-A-93/25573
- US-A- 4 709 013
- CHEMICAL ABSTRACTS, vol. 105, no. 17, 27 October 1986 Columbus, Ohio, US; abstract no. 153558z, NAGANO Y. ET S. TSUKAMOTO: "Preparation of glutathione monoalkyl esters" page 743; column 1; XP002037758 & JP 06 115 870 A (YAMANOUCHI PHARMACEUTICAL CO. LTD. TOKYO, JP) 23 January 1986

## Description

### Background of the Invention

The present invention relates to glutathione (γ-L-glutamyl-L-cysteinylglycine) monoester sulfonate which readily crystallizes and has high stability, and to a process for producing the same.

Glutathione is known to take part in metabolism, transport of metabolites, protection of cells, and the like, and is used as an antidote for liver, a radioprotective agent, an antidote for agricultural use, and so on. Glutathione is produced *in vivo* by biosynthesis from glycine, cysteine and glutamic acid. It is necessary to raise the concentration of glutathione in living cells in order to enhance the cell-protecting activity. However, administration of the above-mentioned amino acids in large quantities raises the concentration of glutathione only within certain limits. Administration of glutathione itself does not result in the desired rise in glutathione concentration: because the administered glutathione is decomposed extracellularly, and after absorption of the constituent amino acids into cells, glutathione is reproduced by intracellular biosynthesis. Thus, it has been difficult to raise the concentration of glutathione in cells beyond certain limits.

On the other hand, it is known that glutathione monoester, unlike glutathione, passes into various types of cells at high concentrations and is converted into glutathione through hydrolysis in the tests using animals [Proc. Natl. Acad. Sci. U.S.A., 80, 5258 (1983)]. Accordingly, glutathione monoester is useful as a readilyabsorbable active form of glutathione which is capable of effectively expressing *in vivo* pharmacological activities of glutathione such as detoxicating activity and radioprotective activity.

With regard to acid addition salts of glutathione monoester formed by ordinary esterification reaction of glutathione, the above-mentioned publication only refers to hydrochloride. However, there is no specific description about the process for producing the salt in the publication. According to US 4,709,013, the hydrochloride cannot be isolated as crystals. Consequently, the purity of the hydrochloride produced is lowered by contamination with glutathione diester which is inevitably formed as a by-product in the esterification process and unreacted glutathione.

US 5,525,628 describes various amino acid addition salts of glutathione monoester and a process for producing the salts. The publication, however, relates to a process for producing the salts from glutathione monoester, and contains no description about a process for producing glutathione monoester itself. That is, in order to obtain the amino acid addition salts of high purity according to the process described in this publication, it is necessary to first obtain glutathione monoester of high purity.

In US 4,709,013, glutathione monoester sulfate and a process for producing the same are described. According to the publication, the sulfate can be obtained as crystals, which facilitates the purification thereof and thus enables the production of glutathione monoester of high purity. However, the publication contains neither description demonstrating the clear crystallizability nor description about the stability upon storage or during the steps of preparation of pharmaceutical compositions. Stability is one of the important properties requisite to a compound which is to be widely used as a drug. There is a report on glutathione monoethyl ester that the precipitate deposited from a sulfuric acid-containing solution with ether is hygroscopic and shows poor stability even when kept at -20°C [Anal. Biochem., 183, 21 (1989)].

JP-A- 61/15870 (Chem. Abstr. vol. 105 no. 17 [27.10.1986] abstract no. 153 558 z) discloses the preparation of glutathione monoalkyl esters by treating protected glutathione monoalkyl esters of formula III (R, R' = protective group for NH₂ and CO₂H, resp.; R", R"' = alkyl) with trifluoromethanesulfonic acid or its mixture with, trifluoroacetic acid.

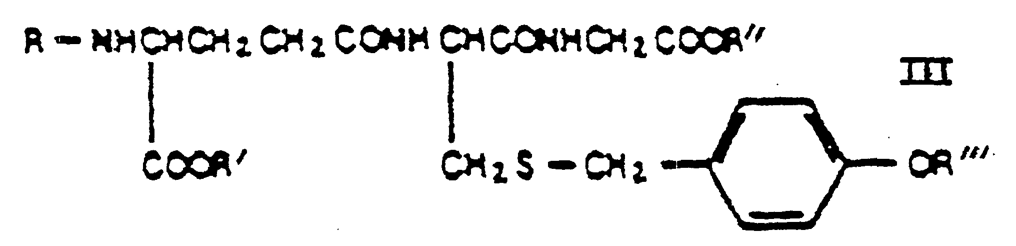

Thus the preparation of Me γ-L-glutamyl-L-cysteinylglycinate trifluoromethanesulfonate salt is disclosed.

An object of the present invention is to provide glutathione monoester sulfonate which readily crystallizes and has high stability, and a simple process for producing the same by which the product of high purity can be obtained in high yields.

### Summary of the Invention

The present invention relates to glutathione monoester sulfonate represented by formula (I): 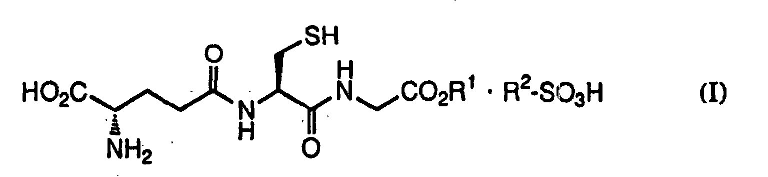 wherein R¹ represents lower alkyl, and R² represents lower alkyl, or substituted or unsubstituted aryl.

The present invention also relates to a process for producing glutathione monoester sulfonate which comprises reacting glutathione with a lower alcohol in the presence of sulfonic acid represented by formula (II):

R²-SO₃H (II)

wherein R² has the same meaning as defined above.

### Brief Description of the Drawings

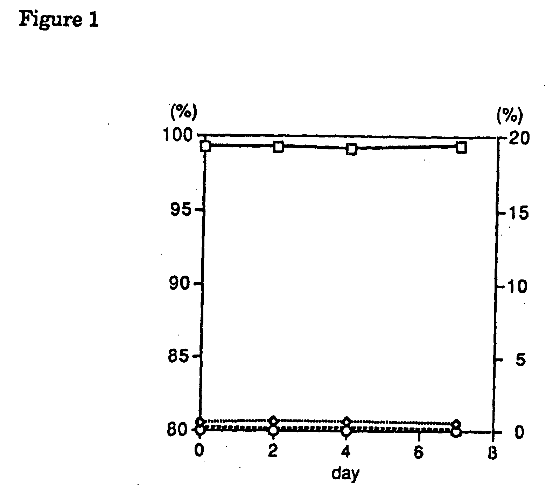

Fig. 1 is a graph showing the stability of glutathione monoethyl ester p-toluenesulfonate on storage. The numbers on the abscissa indicate the passage of time (day), those on the left ordinate indicate the content of glutathione monoethyl ester as determined by high performance liquid chromatography (HPLC) (%), and those on the right ordinate indicate the contents of glutathione, glutathione diethyl ester and other analogues as determined by HPLC (%).

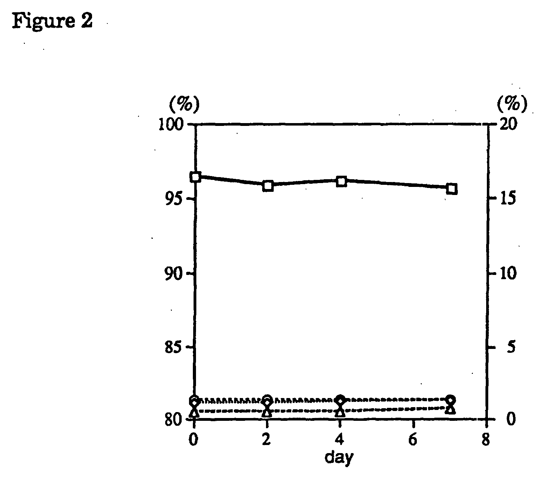

Fig. 2 is a graph showing the stability of glutathione monoethyl ester methanesulfonate on storage. The numbers on the abscissa indicate the passage of time (day), those on the left ordinate indicate the content of glutathione monoethyl ester as determined by HPLC (%), and those on the right ordinate indicate the contents of glutathione, glutathione diethyl ester and other analogues as determined by HPLC (%).

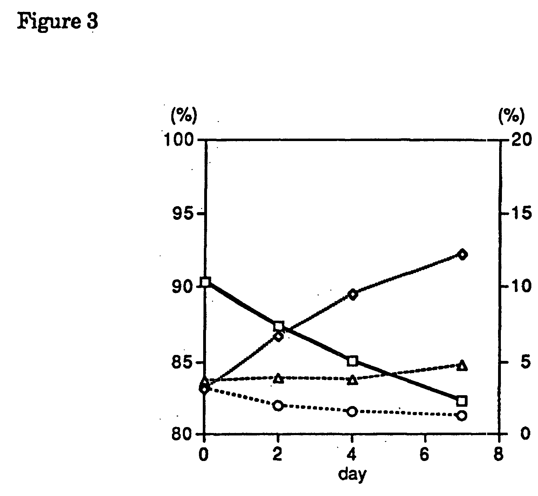

Fig. 3 is a graph showing the stability of glutathione monoethyl ester sulfate on storage. The numbers on the abscissa indicate the passage of time (day), those on the left ordinate indicate the content of glutathione monoethyl ester as determined by HPLC (%), and those on the right ordinate indicate the contents of glutathione, glutathione diethyl ester and other analogues as determined by HPLC (%).

Symbols in the figures:
- ―□― :: Glutathione monoethyl ester
- ―◇― :: Glutathione
- ―○―:: Glutathione diethyl ester
- ―Δ― :: Other analogues

### Detailed Description of the Invention

The compounds represented by formula (I) and formula (II) are hereinafter referred to as Compound (I) and Compound (II), respectively. The same applies to the compounds' formulae with other numbers.

In the definitions of the groups in formula (I), the lower alkyl and the lower alkyl moiety of the lower alcohol mean a straight-chain or branched alkyl group having 1 to .6 carbon atoms, such as methyl, ethyl, propyl, isopropyl, butyl, isobutyl, sec-butyl, tert-butyl, pentyl, isoamyl, neopentyl, 2-pentyl, 3-pentyl, and hexyl. The aryl means phenyl or naphthyl.

The substituted aryl has 1 to 3 independently selected substituents. An example of the substituent is lower alkyl, which has the same meaning as defined above.

The present invention is described in detail below.

Compound (I) can be prepared according to the following reaction step: 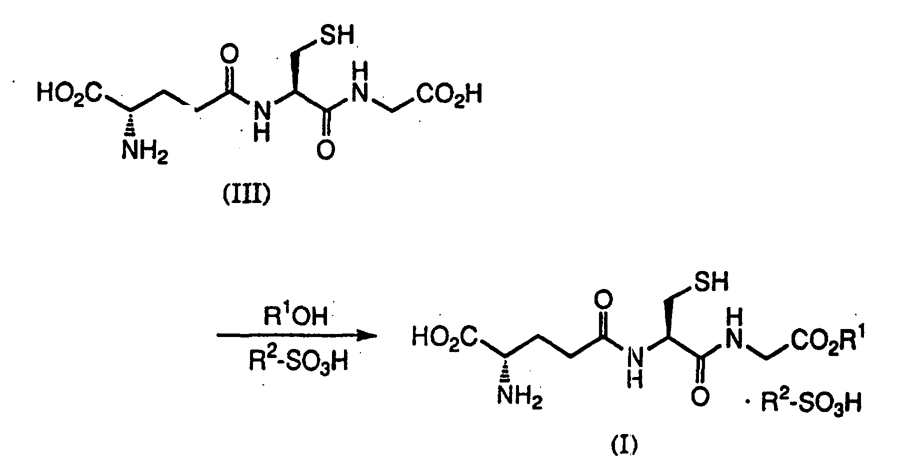 (In the formulae, R¹ and R² have the same meanings as defined above.)

Compound (I) can be obtained by the reaction which proceeds by adding sulfonic acid (II) to a solution or a suspension of glutathione (III) in a large excess of a lower alcohol represented by R¹OH with stirring. As the sulfonic acid (II), those which are commercially available as anhydrides or monohydrates can be used. The sulfonic acid (II) is added in an amount of 1.2 to 3.0 equivalents, preferably 1.5 to 2.0 equivalents, based on glutathione (III). The reaction is carried out at a temperature of - 10°C to the boiling point of the lower alcohol used, preferably at 0 to 50°C, for 5 minutes to 48 hours.

In the above process, usually, the reaction system temporarily affords a homogeneous solution after the addition of sulfonic acid, and as the reaction proceeds, glutathione monoester sulfonate precipitates as crystals. Accordingly, the reaction product can be isolated by merely separating the crystals by filtration. The obtained crystals may be subjected to purification steps such as washing, drying, and recrystallization.

Compound (I) may be in the form of adducts with water or various solvents, which are also within the scope of the present invention.

The stability of glutathione monoester sulfonate of the present invention on storage is illustrated by the following Test Example.

### Test Example 1

The salts obtained in Examples 1 and 2 and Comparative Example 6 were tested for stability on storage. In the test, the salts were allowed to stand under the atmospheric condition at 40°C at ordinary pressure. The results are shown in Figs. 1-3.

As is apparent from the figure, HPLC analysis of the sulfate obtained in Comparative Example 6 revealed considerable decomposition of the salt. Hydrolysis proceeded with the passage of time, and after one week, the contents of glutathione, glutathione diethyl ester, and other analogues were found to be 12.17%, 1.30% and 4.76%, respectively, and the glutathione monoethyl ester content was reduced to 82.29%. This result suggests the difficulty in the storage of glutathione monoester sulfate. In contrast, HPLC analysis of the p-toluenesulfonate and methanesulfonate according to the present invention revealed that the salts were not significantly decomposed. For example, according to the analysis of the p-toluenesulfonate after one week, only glutathione (0.51%) was detected as an analogue, glutathione diethyl ester was not detected, and the glutathione monoethyl ester content was little reduced, i.e. 99.4%. This result demonstrates the high stability of the p-toluenesulfonate and methanesulfonate according to the present invention on storage.

Certain embodiments of the present invention are illustrated in the following Examples, Comparative Examples and Reference Examples.

HPLC was carried out under the following conditions.
Column: YMC AM312 S5 120A ODS
   length, 125 mm; diameter, 6 mm
Mobile phase: acetonitrile/water = 15/85
   (0.05 M phosphate buffer)
Flow rate: 1.0 ml/min.
Detection wavelength: 220 nm
Retention time (min.):
   Glutathione monoethyl ester: 4.1
   Glutathione : 2.7
   Glutathione diethyl ester: 7.2

### Example 1

### Glutathione monoethyl ester p-toluenesulfonate (ethyl γ-L-glutamyl-L-cysteinylglycinate p-toluenesulfonate)

In 4.55 l of ethanol was suspended 130 g of glutathione, and 105 g (2.0 equivalents) of p-toluenesulfonic acid monohydrate was added to the suspension at 25°C with stirring. The suspension became a homogeneous solution in about 10 minutes, and p-toluenesulfonate of glutathione monoethyl ester (ethyl γ-L-glutamyl-L-cysteinylglycinate) started to precipitate as crystals in about 15 hours. After about 20 hours, the reaction mixture was concentrated to 1.95 l. The crystals precipitated were collected by filtration and washed with 0.13 l of ethanol, followed by drying under reduced pressure to give 180 g of a crude p-toluenesulfonate (yield: 84%, HPLC purity: 96.9%). The obtained crude p-toluenesulfonate (180 g) was dissolved in 1.98 l of ethanol at 70°C. The solution was gradually cooled, and then ice cooled to induce crystallization. The crystals precipitated were collected by filtration and washed with 0.18 l of ethanol, followed by drying under reduced pressure to give 153 g of a purified p-toluenesulfonate (yield: 85%, HPLC purity: 99.3%).
Melting Point: 168-170°C

| Elemental Analysis (%): C₁₂H₂₁N₃O₆S·C₇H₈O₃S | | | |
|---|---|---|---|
| Calcd. | C, 44.96; | H, 5.76; | N, 8.28 |
| Found | C, 44.83; | H, 5.63; | N, 7.99 |

IR Absorption Spectrum (KBr, cm⁻¹):
3288, 1747, 1730, 1643, 1535, 1236, 690
Specific Rotation:
[α]_{D}²⁰ = -10.2 (c=10, water)
NMR Spectrum [D₂O, δ (ppm)]:
7.60 (d, J=8.3Hz, 2H), 7.27 (d, J=8.0Hz, 2H), 4.46 (t, J=5.7Hz, 1H), 4.12 (q, J=7.2Hz, 2H), 3.96 (t, J=6.6Hz, 2H), 3.92 (d, J=2.6Hz, 1H), 2.83-2.86 (m, 2H), 2.48-2.54 (m, 2H), 2.30 (s, 3H), 2.09-2.18 (m, 2H), 1.16 (t, J=7.2Hz, 3H)

### Example 2

### Glutathione monoethyl ester methanesulfonate (ethyl γ-L-glutamyl-L-cysteinylglycinate methanesulfonate)

In 9.45 l of ethanol was suspended 450 g of glutathione, and 142 ml (1.5 equivalents) of methanesulfonic acid was added to the suspension at 25°C with stirring. The suspension became a homogeneous solution in about 10 minutes, and methanesulfonate of glutathione monoethyl ester (ethyl γ-L-glutamyl-L-cysteinylglycinate) started to precipitate as crystals in about 20 hours. The reaction was complete in about 24 hours, and the reaction mixture was allowed to stand at room temperature for further 24 hours and then under ice cooling for 24 hours to precipitate crystals. The crystals precipitated were collected by filtration and washed with 0.45 l of ethanol, followed by drying under reduced pressure to give 445 g of a crude methanesulfonate (yield: 70%). The obtained crude methanesulfonate (400 g) was dissolved in 4.40 l of ethanol at 70°C. The solution was gradually cooled, and then ice cooled to induce crystallization. The crystals precipitated were collected by filtration and washed with 0.40 l of ethanol, followed by drying under reduced pressure to give 317 g of a purified methanesulfonate (yield: 79%).
Melting Point: 149-151°C

| Elemental Analysis (%): C₁₂H₂₁N₃O₆S·CH₄O₃S | | | |
|---|---|---|---|
| Calcd. | C, 36.19; | H, 5.84; | N, 9.74 |
| Found | C, 36.25; | H, 5.69; | N, 9.45 |

IR Absorption Spectrum (KBr, cm⁻¹):
3337, 1747, 1730, 1637, 1539, 1232
NMR Spectrum [D₂O, δ (ppm)]:
4.46 (t, J=5.7Hz, 1H), 4.12 (q, J=7.2Hz, 2H), 3.99 (t, J=6.7Hz, 2H), 3.93 (d, J=2.6Hz, 1H), 2.84-2.86 (m, 2H), 2.49-2.55 (m, 2H), 2.71 (s, 3H), 2.11-2.19 (m, 2H), 1.17 (t, J=7.2Hz, 3H)

### Comparative Example 1

### Glutathione monoethyl ester sulfate (ethyl γ-L-glutamyl-L-cysteinylglycinate sulfate)

In 40 ml of ethanol was suspended 9.92 g of glutathione, and 2.4 ml (1.4 equivalents) of sulfuric acid was added dropwise to the suspension with stirring under ice cooling. The suspension became a homogeneous solution in about 30 minutes. Precipitation of sulfate of glutathione monoethyl ester (ethyl γ-L-glutamyl-L-cysteinylglycinate) as crystals was not observed after stirring at room temperature for 24 hours. The solution was further stirred under ice cooling for 24 hours, but the sulfate was not precipitated as crystals, either.

### Comparative Example 2

### Glutathione monoethyl ester sulfate (ethyl γ-L-glutamyl-L-cysteinylglycinate sulfate)

In 40 ml of ethanol was suspended 9.92 g of glutathione, and 2.4 ml (1.4 equivalents) of sulfuric acid was added dropwise to the suspension with stirring under ice cooling. The suspension became a homogeneous solution in about 30 minutes. Precipitation of sulfate of glutathione monoethyl ester (ethyl γ-L-glutamyl-L-cysteinylglycinate) as crystals was not observed after stirring at room temperature for 24 hours. The solution was further stirred under ice cooling for 43 hours, but the sulfate was not precipitated as crystals, either.

### Comparative Example 3

### Glutathione monoethyl ester sulfate (ethyl γ-L-glutamyl-L-cysteinylglycinate sulfate)

In 40 ml of ethanol was suspended 9.92 g of glutathione, and 2.4 ml (1.4 equivalents) of sulfuric acid was added dropwise to the suspension with stirring under ice cooling. The suspension became a homogeneous solution in about 30 minutes. Precipitation of sulfate of glutathione monoethyl ester (ethyl γ-L-glutamyl-L-cysteinylglycinate) as crystals was not observed after stirring at room temperature for 24 hours. The solution was further stirred under ice cooling for 48 hours, but the sulfate was not precipitated as crystals, either. Then, the solution was concentrated to 25 ml, followed by addition of 10 ml of diethyl ether. However, precipitation of crystals was not still observed.

### Comparative Example 4

### Glutathione monoethyl ester sulfate (ethyl γ-L-glutamyl-L-cysteinylglycinate sulfate)

In 260 ml of ethanol was suspended 49.6 g of glutathione, and 12 ml (1.4 equivalents) of sulfuric acid was added dropwise to the suspension with stirring under ice cooling. The suspension became a homogeneous solution in about 30 minutes. Precipitation of sulfate of glutathione monoethyl ester (ethyl γ-L-glutamyl-L-cysteinylglycinate) as crystals was not observed after stirring at room temperature for 24 hours. The solution was further stirred under ice cooling for 24 hours, but the sulfate was not precipitated as crystals, either.

### Comparative Example 5

### Glutathione monoethyl ester sulfate (ethyl γ-L-glutamyl-L-cysteinylglycinate sulfate)

In 400 ml of ethanol was suspended 99.2 g of glutathione, and 24 ml (1.4 equivalents) of sulfuric acid was added dropwise to the suspension with stirring under ice cooling. The suspension became a homogeneous solution in about 30 minutes. Precipitation of sulfate of glutathione monoethyl ester (ethyl γ-L-glutamyl-L-cysteinylglycinate) as crystals was not observed after stirring at room temperature for 24 hours. The solution was further stirred under ice cooling for 24 hours, but the sulfate was not precipitated as crystals, either.

### Comparative Example 6

### Glutathione monoethyl ester sulfate (ethyl γ-L-glutamyl-L-cysteinylglycinate sulfate)

In 260 ml of ethanol was suspended 49.6 g of glutathione, and 12 ml (1.4 equivalents) of sulfuric acid was added dropwise to the suspension with stirring under ice cooling. The suspension became a homogeneous solution in about 30 minutes. Precipitation of sulfate of glutathione monoethyl ester (ethyl γ-L-glutamyl-L-cysteinylglycinate) as crystals was not observed after stirring at room temperature for 24 hours. The solution was further stirred under ice cooling for 15 hours, but the sulfate was not precipitated as crystals, either. Then, the sulfate of glutathione monoethyl ester obtained in Reference Example was added to the solution as seed crystals. There appeared white turbidity in about 3 hours, and a large amount of crystals precipitated in about 6 hours. The crystals precipitated were collected by filtration and washed with 100 ml of ethanol, followed by drying under reduced pressure to give 22.5 g of a crude sulfate (yield: 36.3%). The obtained crude sulfate (5.00 g) was dissolved in a mixture of 75 ml of ethanol and 75 ml of water under heating, and the solution was gradually cooled to induce crystallization. The crystals precipitated were collected by filtration and washed with 15 ml of ethanol, followed by drying under reduced pressure to give 16.0 g of the desired compound (yield: 71.0%).

### Comparative Example 7

### Glutathione monoethyl ester sulfate (ethyl γ-L-glutamyl-L-cysteinylglycinate sulfate)

In 400 ml of ethanol was suspended 99.2 g of glutathione, and 24 ml (1.4 equivalents) of sulfuric acid was added dropwise to the suspension with stirring under ice cooling. The suspension became a homogeneous solution in about 30 minutes. Precipitation of sulfate of glutathione monoethyl ester (ethyl γ-L-glutamyl-L-cysteinylglycinate) as crystals was not observed after stirring at room temperature for 24 hours. Then, the sulfate of glutathione monoethyl ester obtained in Reference Example was added to the solution as seed crystals, followed by stirring under ice cooling for 24 hours. The crystals precipitated were collected by filtration and washed with 200 ml of ethanol, followed by drying under reduced pressure to give 39.5 g of a crude sulfate (yield: 31.8%).

### Reference Example

### (Desalting and preparation of sulfate: preparation of glutathione monoethyl ester and its sulfate having high purity)

In 0.5 l of water was dissolved 50.0 g of the p-toluenesulfonate obtained in Example 1, and the solution was charged into a column packed with 200 ml of WA30 (weakly basic ion-exchange resin; Mitsubishi Chemical Corporation) previously treated with a 4% aqueous solution of acetic acid. Elution was carried out with water to give 1.0 l of a fraction containing the desired substance. Concentration of this fraction gave 33.0 g of glutathione monoethyl ester of high purity in the free state (yield: 94.6%, HPLC purity: 99.3%). The obtained glutathione monoethyl ester (5.00 g) was suspended in a mixture of 100 ml of ethanol and 10 ml of water, and 0.9 ml (1.2 equivalents) of sulfuric acid was added dropwise to the suspension with stirring under ice cooling. The suspension became a homogeneous solution in about 30 minutes. The solution was further stirred, whereby a sulfate of glutathione monoethyl ester (ethyl γ-L-glutamyl-L-cysteinylglycinate) precipitated as crystals. The crystals precipitated were collected by filtration and dried under reduced pressure to give 1.13 g of the desired pure sulfate (yield: 19.2%).

The results of HPLC analysis of the salts obtained in Examples 1 and 2 and Comparative Example 6 are shown in Table 1.

**Table 1**

| | Content determined by HPLC (%) | | | |
|---|---|---|---|---|
| | GSH-Et | GSH | GSH-Et₂ | Others |
| p-Toluenesulfonate | 99.3 | 0.54 | N.D. | 0.16 |
| Methanesulfonate | 96.4 | 1.12 | 1.30 | 1.18 |
| Sulfate | 90.3 | 3.12 | 3.25 | 3.73 |
| GSH-Et: Glutathione monoethyl ester GSH: Glutathione GSH-Et₂: Glutathione diethyl ester Others: Other analogues N.D.: Not detected | | | | |

## Claims

1. Glutathione monoester sulfonate represented by formula (I) : 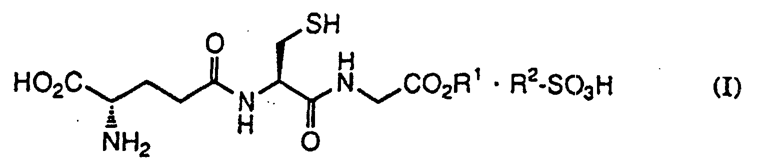 wherein R¹ represents lower alkyl, and R² represents lower alkyl, or substituted or unsubstituted aryl.

2. Glutathione monoester sulfonate according to Claim 1, wherein R¹ is ethyl.

3. Glutathione monoester sulfonate according to Claim 2, wherein R² is methyl.

4. Glutathione monoester sulfonate according to Claim 2, wherein R² is p-tolyl.

5. A process for producing glutathione monoester sulfonate which comprises reacting glutathione with a lower alcohol in the presence of sulfonic acid represented by formula (II):
R²-SO₃H (II)
wherein R² has the same meaning as defined above.

6. A process according to Claim 5, wherein the lower alcohol is ethanol.

7. A process according to Claim 6, wherein R² is methyl.

8. A process according to Claim 6, wherein R² is p-tolyl.

## Patentansprüche

1. Glutathionmonoestersulfonat der Formel (I) 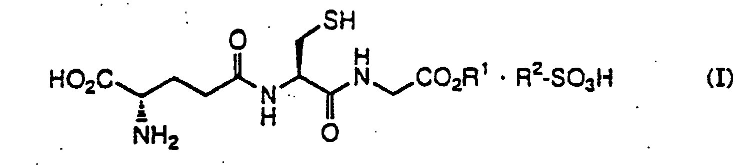 wobei R¹ einen Niederalkylrest und R² einen Niederalkyl- oder substituierten oder unsubstituierten Arylrest darstellt.

2. Glutathionmonoestersulfonat nach Anspruch 1, wobei R¹ eine Ethylgruppe ist.

3. Glutathionmonoestersulfonat nach Anspruch 2, wobei R² eine Methylgruppe ist.

4. Glutathionmonoestersulfonat nach Anspruch 2, wobei R² eine p-Tolylgruppe ist.

5. Verfahren zur Herstellung von Glutathionmonoestersulfonat, das die Umsetzung von Glutathion mit einem niederen Alkohol in Gegenwart einer Sulfonsäure der Formel (II) umfasst
R²-SO₃H (II),
wobei R² die gleiche Bedeutung hat wie vorstehend definiert.

6. Verfahren nach Anspruch 5, wobei der niedere Alkohol Ethanol ist.

7. Verfahren nach Anspruch 6, wobei R² Methyl ist.

8. Verfahren nach Anspruch 6, wobei R² p-Tolyl ist.

## Revendications

1. Sulfonate de monoester de glutathion, représenté par la formule (I) : 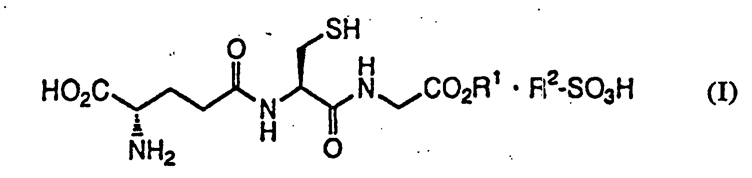 dans laquelle R¹ représente un groupe alkyle inférieur et R² représente un groupe alkyle inférieur ou un groupe aryle substitué ou non.

2. Sulfonate de monoester de glutathion, conforme à la revendication 1, dans lequel R¹ représente un groupe éthyle.

3. Sulfonate de monoester de glutathion, conforme à la revendication 2, dans lequel R² représente un groupe méthyle.

4. Sulfonate de monoester de glutathion, conforme à la revendication 2, dans lequel R² représente un groupe p-tolyle.

5. Procédé de préparation d'un sulfonate de monoester de glutathion, qui comporte le fait de faire réagir du glutathion avec un alcool inférieur, en présence d'un acide sulfonique représenté par la formule (H) :
R²-SO₃H (II)
dans laquelle R² a la même signification que celle indiquée ci-dessus.

6. Procédé conforme à la revendication 5, dans lequel l'alcool inférieur est de l'éthanol.

7. Procédé conforme à la revendication 6, dans lequel R² représente un groupe méthyle.

8. Procédé conforme à la revendication 6, dans lequel R² représente un groupe p-tolyle.
